# EUROPÄISCHE PATENTSCHRIFT

(11) **EP 3 818 966 B1**
(45) Veröffentlichungstag und Bekanntmachung des Hinweises auf die Patenterteilung: **23.10.2024**
(21) Anmeldenummer: 20205344.3
(22) Anmeldetag: 03.11.2020
(51) Int. Cl.: A61F 7/00

(54) **MEDIZINISCHES TEMPERIERGERÄT UND HERSTELLUNGSVERFAHREN**
MEDICAL TEMPERATURE CONTROL DEVICE AND METHOD OF MANUFACTURING THE SAME
APPAREIL MÉDICAL DE MISE EN TEMPÉRATURE ET PROCÉDÉ DE FABRICATION

(30) Priorität: 11.11.2019 DE 202019004595 U
(43) Veröffentlichungstag der Anmeldung: 12.05.2021
(73) Patentinhaber: Hilotherm Holding AG, 6317 Oberwil bei Zug (CH)
(72) Erfinder: Stegmann, Christian, 87545 Burgberg im Allgäu (DE)
(74) Vertreter: Wallinger, Michael

(56) Entgegenhaltungen:
- EP-A1- 3 192 477
- DE-A1- 102016 000 345
- US-B1- 10 350 108

## Beschreibung

Die Erfindung betrifft ein medizinisches Temperiergerät und ein Verfahren zu dessen Herstellung.

Die Heilwirkung von Wärme- und Kältebehandlungen für den menschlichen und tierischen Körper ist schon seit langem bekannt. Während einfache Temperierungseinrichtungen, wie Eisbeutel oder Wärmeflaschen, als Hausmittel nach wie vor Verwendung finden, hat man in der professionellen medizinischen Therapie erkannt, dass sich der Heilerfolg solcher Maßnahmen bedeutend verbessert, wenn man den betroffenen Körperbereich mit einer bestimmten, meist konstanten, Temperatur versorgt. Wird das Gewebe beispielsweise lokal abgekühlt, tritt an dieser Stelle eine Minderung der Durchblutung und des lokalen Stoffwechsels ein. Beides wirkt sich entzündungshemmend und abschwellend aus. Eine solche therapeutische Maßnahme, die sehr erfolgreich bei der Behandlung von traumatisierten oder entzündeten Gelenken eingesetzt wird, ist z. B. eine gleichmäßige Temperierung auf 15°C.

Um diese Temperierung zu erreichen, wird auf die betroffenen Körperbereiche eine Temperier-Manschette aufgelegt, die eine Vielzahl von Strömungswegen für Wasser enthält. Diese Manschette wird dann von einem Temperiergerät der hier in Rede stehenden Art (HILOTHERM Professionell Gerät der Firma Hilotherm GmbH) mit temperiertem Wasser versorgt, so dass die gewünschte Temperatur über die Behandlungszeit im Wesentlichen konstant gehalten bzw. an einen gewünschten Temperaturverlauf angepasst werden kann.

Es zeigte sich nun in klinischen Studien der Erfinder, dass die medizinische Therapiewirkung bei bestimmten Anwendungen verbessert werden kann, indem auch die Einstellung einer Temperatur tiefer als 15°C ermöglich wird.

Die vorliegende Erfindung stellt sich deshalb die Aufgabe, den Temperaturbereich des genannten Temperiergerätes auf niedrigere Temperaturen zu erweitern.

Diese Aufgabe wird erfindungsgemäß durch eine Temperiergerät gemäß Patentanspruch 1 und das Verfahren zur Herstellung eines Temperiergerätes gemäß Anspruch 15 gelöst. Zu bevorzugende Weiterbildungen der Erfindung sind Gegenstände der Unteransprüche.

Ein medizinisches Temperiergerät der vorliegend betrachteten Art zur Temperierung eines Fluids hat wenigstens ein Gehäuse, wenigstens eine Fluidversorgungseinrichtung, durch welche ein Fluid in dem Gehäuse bereitgestellt wird, wenigstens eine im Gehäuse angeordnete Temperiereinrichtung, die das durch das Gehäuse strömende Fluid temperiert, wenigstens eine Fluidabströmungseinrichtung, durch welche das Fluid mit einer im Wesentlichen vorbestimmten Temperatur aus dem Gehäuse herausgeführt wird, und wenigstens eine im Gehäuse angeordnete Pumpeneinrichtung, welche das durch das Gehäuse strömende Fluid mit Druck beaufschlagt.

Unter einer Fluidversorgungseinrichtung wird dabei eine Einrichtung verstanden, die das Vorhandensein des Fluids während des Betriebs des Temperiergerätes in stets ausreichender Menge sicherstellt oder es dem Benutzer des Temperiergerätes ermöglicht, dies zu tun, insbesondere durch geeignete Mittel zur Bevorratung und/oder Nachfüllung des Fluids Die Fluidversorgungseinrichtung kann zu diesem Zweck beispielsweise entsprechende Vorratsbehälter zur Speicherung des Fluids aufweisen. Dabei können die Vorratsbehälter als Tanks zur Aufnahme des Fluids ausgebildet sein, wobei diese innerhalb oder auch außerhalb des Gehäuses angebracht sein können. Die Fluidversorgungseinrichtung umfasst daher im Falle eines außerhalb des Gehäuses angebrachten Vorratsbehälters die Zu- und Ableitungen, die für ein hinein und herausströmen des Fluids aus den Vorratsbehältern heraus und in diese zurück notwendig sind.

Das Fluid ist vorzugsweise Wasser, insbesondere destilliertes Wasser, kann aber auch Leitungswasser, Wasser mit bestimmten Zusatzstoffen oder ein anderes Fluid als Wasser, insbesondere Öl, sein. Das Fluid kann auch als zwei- bzw. mehrphasiges Fluid ausgebildet sein. Darunter sind Gemische unterschiedlicher, beispielsweise getrennt voneinander aufbewahrter Fluide zu verstehen, beispielsweise zur Verbesserung und Förderung der Heilwirkung des Behandlungsverfahrens. Dabei werden die Fluide in beispielsweise unterschiedlichen Vorratsbehältern aufbewahrt und dann während des Betriebes des Therapiergerätes wie ein einzelnes Fluid in dem Fluidkreislauf gefördert und dabei durchmischt.

Unter einer Fluidabströmungseinrichtung wird eine Einrichtung verstanden, durch welche das Fluid aus dem Gehäuse heraus und in Richtung der zu behandelnden Körperfläche kontrolliert abströmt. Die Fluidabströmungseinrichtung umfasst beispielsweise eine als flexiblen Schlauch ausgebildete Leitung, durch welchen das abströmende Fluid von dem Gehäuse weg, zu der zu behandelnden Körperfläche hin geführt wird. Weiter umfasst die Fluidabströmungseinrichtung ein Kupplungsstück, welches fest mit dem Gehäuse verbunden ist. Der Schlauch ist mit dem mechanischen Kupplungsstück fluidleitend verbunden und damit über das Kupplungsstück mechanisch an dem Gehäuse fixiert. Der Schlauch ist dabei aufsteckbar fluidleitend an das Kupplungsstück gekoppelt, so dass das Fluid durch das Kupplungsstück hindurch nach Außen abströmen kann.

Unter einer Pumpeneinrichtung wird mindestens eine Pumpe zum Fördern des Fluids und die zur Regelung der mindestens einen Pumpe notwenige Elektronik verstanden. Die Pumpe kann beispielsweise eine Flügelradpumpe sein. Die Pumpe fördert das Fluid, so dass das Fluid in einem Kreislauf zirkuliert und Wärmeenergie zwischen dem zirkulierenden Fluid und der ersten und der zweiten Temperiereinheit ausgetauscht wird und eine vom Benutzer voreingestellte Temperatur der Fluids gehalten wird.

Unter einer Temperiereinrichtung wird eine Einrichtung zum Heizen und/oder Kühlen des Fluids verstanden, vorzugsweise durch Umwandlung von elektrischer Energie in Wärmeenergie bzw. durch Verwendung von elektrischer Energie zum Entziehen von Wärmeenergie und vorzugsweise mit einem Wärmetauscher zur Übertragung von erzeugter Wärme auf das Fluid bzw. zum Entziehen von Wärme aus dem Fluid. Dabei weist die wenigstens eine Temperiereinrichtung wenigstens eine erste Temperiereinheit und wenigstens eine zweite Temperiereinheit auf, welche jeweils wenigstens ein thermoelektrisches Element, insbesondere wenigstens ein Peltier-Element, wenigstens ein von Fluid durchströmbares Wärmetauscherelement zur Übertragung von Wärme auf das oder von dem Fluid und wenigstens einen Kühlkörper aufweisen
Unter einer ersten, bzw. zweiten Temperiereinheit ist jeweils eine Temperiereinheit zu verstehen, die als eine Wärme - bzw. Kältequelle ausgebildet ist und das im bzw. durch das Temperiergerät zirkulierende Fluid auf eine von einem Benutzer vorbestimmte Temperatur regelt, wobei jede der Temperiereinheiten voneinander unabhängig arbeitet, d.h. unabhängig voneinander das Fluid kühlen oder erwärmen kann. Das Fluid durchströmt dabei das Wärmetauscherelement der Temperiereinheit, wobei ein Wärmetausch in Form eines Wärmetransportes stattfindet. Die Richtung des Wärmetransportes ergibt sich aus der Temperatur des Fluids relativ zu der Temperatur des Wärmetauschers, welcher durch ein Thermoelement, beispielsweise durch ein Peltier Element, in seiner Temperatur kontrolliert wird. Das Peltier Element regelt dabei über mindestens einen Temperatur Sensor mittels einer elektronischen Regelschleife zwischen einer Ist - und einer von einem Benutzer voreingestellten Soll - Temperatur des Fluids, so dass die Soll - Temperatur des Fluids möglichst genau erzielt und beibehalten, d.h. durch die Regelung konstant gehalten wird. Im Falle einer Kühlung des Fluids wird dabei Wärmeenergie von dem Fluid über den Wärmetauscher zu dem Thermoelement transportiert, d.h. dem Fluid entzogen wodurch sich dieses abkühlt. Die damit verbundene Erwärmung des Thermoelementes wird durch entsprechende elektrische Leistung, welche die Kühlwirkung des Thermoelementes erhöht, mittels des elektrischen Regelkreises korrigiert. Zusätzlich wird der damit verbundene Wärmeüberschuss am Thermoelement durch Wärmeabtransport an einem am Thermoelement angebrachten Kühlkörper abgeführt. Die weitere Abfuhr der Wärme von dem Kühlkörper an die Umgebung kann dabei mittels eines kühlenden Luftstroms, der am Kühlkörper entlang oder durch diesen hindurch strömt, effizient umgesetzt werden.

Daher ist das medizinische Temperiergerät in der Lage einen Luftstrom zu erzeugen.

Erfindungsgemäß wird dabei ein erster Luftstrom auf den wenigstens einen ersten Kühlkörper der ersten Temperiereinheit gerichtet und ein zweiter Luftstrom auf den wenigstens einen zweiten Kühlkörper der zweiten Temperatureinheit gerichtet.

Bei verletztem bzw. traumatisiertem Gewebe behindern Ödeme und Einblutungen die Versorgung mit Sauerstoff und Nährstoffen, was zu Durchblutungsstörungen und einer entzündungsbedingten Überwärmung führt. Wird eine für den geschädigten Gewebebereich genau passende Temperatur eingesetzt, kann der Stoffwechsel in den Zustand versetzt werden, abgestorbene Zellen und Proteine zu 'entsorgen', wodurch sich der Heilungsprozess beschleunigt. Dazu wird die Gewebe-Temperatur je nach Verletzungsart auf einen bestimmten Wert abgesenkt oder erhöht.

Mit der Erfindung wird erreicht, dass der Anwendungsbereich des Wärmeheilverfahrens auf weitere Gewebebereiche ausgedehnt werden kann bzw. geeignetere Kühltemperaturen für zu behandelnde Gewebebereiche ausgewählt werden können, so dass ein Heilungsprozess beschleunigt wird.

Die Erfindung wird vorliegend am Beispiel eines Temperiergerätes zur Verwendung mit einer wasserdurchströmten Temperier-Manschette zum Auflegen auf die betroffenen Körperbereiche des Patienten beschrieben. Dies stellt jedoch keine Einschränkung dar; das erfindungsgemäße medizinische Temperiergerät lässt sich auch für andere Anwendungen einsetzen, beispielsweise zur Verwendung mit einem Temperierkörper, welcher in eine Körperöffnung des Patienten eingeführt wird.

Die vorliegende Erfindung bedeutet eine Weiterentwicklung der bisher verwendeten Konstruktion für derartige Temperiergeräte.

Temperiereinheiten, welche ein thermoelektrisches Element, ein Wärmetauscherelement und einen Kühlkörper aufweisen, werden bereits als fertige Module, sogenannte "Liquidto-Air-Module", am Markt angeboten. Hierbei werden als thermoelektrische Elemente vorzugsweise die aus bestimmten Halbleitern bestehenden, sogenannten "Peltier - Elemente" eingesetzt. Die Temperierleistung eines thermoelektrischen Elements beruht darauf, dass beim Anlegen einer Spannung an die elektrischen Anschlüsse des Elements zwischen gegenüberliegenden Oberflächen des Elements eine Temperaturdifferenz erzeugt wird, d. h. es wird Wärme von der einen zu der anderen Oberfläche transportiert. Es ist daher zweckmäßig, auf einer dieser Oberflächen das Wärmetauscherelement und auf der anderen Oberfläche den Kühlkörper anzuordnen. Entsprechend der Polung der Peltier-Elemente kann die Kühl- bzw. Heizfunktion der gegenüberliegenden Platten getauscht werden.

Im Folgenden wird der Einsatz des medizinischen Temperiergerätes für eine Kälteanwendung betrachtet, d. h. die vorbestimmte Temperatur, mit der das Fluid aus dem Gehäuse herausgeführt wird, soll geringer sein als die Ausgangstemperatur, mit der das Fluid von der Fluidversorgungseinrichtung bereitgestellt wird. In diesem Fall wird das thermoelektrische Element so geschaltet, dass Wärme von der zum Wärmetauscherelement hin orientierten Oberfläche zu der zum Kühlkörper hin orientierten Oberfläche transportiert wird. Auf diese Weise kann dem Fluid über das Wärmetauscherelement an der entsprechenden Oberfläche des thermoelektrischen Elements Wärme entzogen und diese Wärme auf der anderen Oberfläche des thermoelektrischen Elements an den Kühlkörper abgegeben werden, so dass das Fluid abgekühlt und der Kühlkörper erwärmt wird.

Für die Leistung der gesamten Temperiereinheit ist entscheidend, in welchem Maße die im Kühlkörper entstehende Wärme wieder in die Umgebung abgeführt wird, da hiervon die am thermoelektrischen Element entstehende Temperaturdifferenz abhängt.

In dem bisherigen Temperiergerät gemäß der EP 3 192 477 A1 ist eine kompakte Anordnung vorgesehen, wobei die Kühlkörper der beiden Temperiereinheiten einander zugewandt sind, d.h. die Kühlflächen der Kühlkörper sind übereinander gestapelt und die Kühlkörper sind dabei spiegelsymmetrisch zueinander angeordnet. Insbesondere wenn die Kühlkörper Rippen aufweisen, sind die Kühlkörper der ersten bzw. der zweiten Temperiereinheit so übereinander gestapelt, dass benachbarte Stege einander angrenzender Kühlrippen des ersten Kühlkörpers entlang benachbarter Stege einander angrenzender Kühlrippen des zweiten Kühlkörpers verlaufen, so dass jeweils durch zwei benachbarte Kühlrippen des ersten Kühlkörpers und benachbarte Kühlrippen des zweiten Kühlkörpers ein gemeinsamer Kühlkanal gebildet wird, durch welchen ein Luftstrom geleitet werden kann.

Dadurch wird vorteilhaft eine kompakte Bauform des Temperiergerätes erzielt, da durch den gemeinsamen Kühlkanal nur ein einziger Luftstrom zur Kühlung zweier Kühlkörper zum Einsatz kommt. Dadurch lassen sich zusätzlich die Herstellungskosten senken.

Um den Temperaturbereich des genannten Temperiergerätes auf niedrigere Temperaturen zu erweitern schlägt die Erfindung nun vor, die Anordnung der Kühlkörper so abzuändern, dass ein erster Luftstrom auf den Kühlkörper der ersten Temperiereinheit gerichtet ist und ein zweiter Luftstrom auf den Kühlkörper der zweiten Temperiereinheit gerichtet ist.

In der bisherigen Anordnung findet keine Aufteilung des Luftstroms statt. Stattdessen wird ein durch beide Kühlkörper strömender gemeinsamer Luftstrom erzeugt. Beispielsweise durch eine erste Ventilationseinrichtung, welche Luft von einer Seite durch die von beiden Kühlkörpern gemeinsam gebildeten Kühlkanale drückt und an der entgegengesetzten Seite der Kühlkanäle, d.h. dort wo die zugeführte Luft aus den Kanälen ausströmt, durch eine zweite Ventilationseinrichtung aus den Kanälen absaugt.

Bei erfindungsgemäßer Anordnung werden stattdessen die jeweiligen Kühlkörper der ersten und der zweiten Temperiereinheit getrennt voneinander über unabhängige Luftströme gekühlt.

Ein Abändern der Anordnung der Kühlkörper auf diese Weise bewirkt, dass die jeweiligen Kühlkörper der ersten bzw. der zweiten Temperiereinheit jeweils unabhängig voneinander von einem kühlenden Luftstrom getroffen werden. Damit wird eine Verbesserung der Kühlung der ersten und der zweiten Temperiereinheit erzielt.

Bei erfindungsgemäßer Anordnung ist damit die zu kühlende Fläche pro Luftstrom reduziert, wodurch sich die Kühlleistung verbessert.

Das medizinische Temperiergerät ist eingerichtet, diesen Luftstrom zu erzeugen. In einer besonders bevorzugten Ausführungsform weisen die erste Temperiereinheit und die zweite Temperiereinheit jeweils wenigstens eine Ventilationseinrichtung auf.

In einer bevorzugten Ausführungsform des medizinischem Temperiergerätes weist der erste Kühlkörper der ersten Temperiereinheit einen Bereich mit Kühlrippen auf und der Bereich mit Kühlrippen der ersten Temperiereinheit wird von dem ersten Luftstrom durchströmt wird, und der zweite Kühlkörper der zweiten Temperiereinheit weist einen Bereich mit Kühlrippen auf und der Bereich mit Kühlrippen der zweiten Temperiereinheit wird von dem zweiten Luftstrom durchströmt wird.

Damit wird die Oberfläche des zu kühlenden Volumens des Kühlkörpers vergrößert und Wärme kann besser von dem Kühlkörper an die Umgebung abgegeben bzw. durch einen Luftstrom, der die Kühlrippen umströmt, von dem Kühlkörper abtransportiert werden.

In einer bevorzugten Ausführungsform verläuft der erste Luftstrom im Wesentlichen parallel zu den Ebenen, in denen sich die jeweiligen Kühlrippen des ersten Kühlkörpers der ersten Temperiereinheit (7) erstrecken und welche senkrecht zu der Ebene angeordnet sind, die sich durch das unmittelbar flächige aneinandergrenzen von dem ersten Thermoelement und dem ersten Kühlkörper ergibt und entsprechend verläuft der zweite Luftstrom im Wesentlichen parallel zu den Ebenen, in denen sich die jeweiligen Kühlrippen des zweite Kühlkörpers der zweiten Temperiereinheit erstrecken und welche senkrecht zu der Ebene angeordnet sind, die sich durch das unmittelbar flächige aneinandergrenzen von dem zweitem Thermoelement und dem zweiten Kühlkörper ergibt. Der genannte erste bzw. zweite Luftstrom bezeichnet insbesondere dessen Abschnitt vor bzw. unmittelbar vor dem Eintritt in den jeweiligen Kühlkörper. Die Richtung des ersten Luftstroms ist insbesondere senkrecht zu einer ersten Bodenplatte des ersten Kühlkörpers gerichtet. Die Richtung des zweiten Luftstroms ist insbesondere senkrecht zu einer zweiten Bodenplatte des zweiten Kühlkörpers gerichtet.

Damit lässt sich ein starker erster und zweiter Luftstrom durch die Kühlschlitze der jeweiligen Kühlkörper der Temperiereinheiten realisieren und somit eine gute Wärmeabfuhr von den Kühlkörpern erzielen. Eine Anströmung in Richtung senkrecht zur Bodenplatte und damit insbesondere im Wesentlichen parallel zu den Ebenen in denen sich die jeweiligen Kühlrippen erstrecken hat zudem den Vorteil, Leebereiche, d.h. von der Strömung abgeschattete, turbulente Bereiche zu vermeiden, da in diesen, turbulenten Strömungsbereichen die Kühlung aufgrund der reduzierten Wärmeabfuhr vermindert ist.

Die Kühlrippen des Kühlkörpers der ersten Temperiereinheit und/oder die Kühlrippen des Kühlkörpers der zweiten Temperiereinheit sind dabei vorzugsweise im sogenannten "Skived-Fin"-Verfahren hergestellt.

Bei diesem Verfahren wird ein massiver, annähernd quaderförmiger Metallblock mit einer Vielzahl von schräg zur Oberfläche gerichteten Schnitten in parallelen Ebenen bis zu einer bestimmten Tiefe, insbesondere bis kurz vor die gegenüberliegende Seite des Blockes, versehen. Sodann werden die hierbei zwischen benachbarten Schnitten entstandenen Metallscheiben aufgerichtet, so dass sie eng benachbarte, voneinander nur geringfügig beabstandete Rippen bilden, welche senkrecht zur gegenüberliegenden Seitenfläche des Blockes stehen. Diese gegenüberliegende Seitenfläche bildet eine durchgehende, ebene Oberfläche des Kühlkörpers, an welcher das zu kühlende Element befestigt wird.

Auf diese Weise lässt sich in einem nicht-spanenden und nicht-abtragenden Herstellungsverfahren ein Kühlkörper aus einem Stück und damit ohne Materialübergänge mit einer Vielzahl von eng nebeneinander stehenden Kühlrippen fertigen, wodurch sich eine entsprechend hohe Kühlleistung des Kühlkörpers erreichen lässt.

Eine weiter bevorzugte Ausführungsform zeichnet sich dadurch aus, dass die Kühlkörper der ersten und der zweiten Temperiereinheit aus Kupfer gefertigt sind.

Kupfer hat einen im Verhältnis zu anderen Metallen, wie z.B. Aluminium hohen Wärmeleitungskoeffizienten von z.B. 240 - 380 W/(mK) (AI: 236 W/(mK)). Damit lässt sich die Wärmeabfuhr zwischen Wärmetauscher und Kühlkörper verbessern. Dies steigert Kühleffizienz der Temperiereinrichtung.

In einer besonders bevorzugten Ausführungsform, sind die erste Temperiereinheit und die zweite Temperiereinheit im Wesentlichen spiegelsymmetrisch zueinander angeordnet.

Eine spiegelsymmetrische Anordnung der Temperiereinheiten zueinander erlaubt es, die Lüfter in Richtung der Gehäusewände des medizinischen Temperiergerätes zu orientieren. Damit lässt sich mit geringem konstruktivem Aufwand ein definierter Ansaugbereich der Lüfter durch Öffnungen in gegenüberliegenden Gehäusewänden oder benachbarten Wandabschnitten des Gehäuses des Therapiegerätes festlegen.

In einer bevorzugten Ausführungsform des medizinischen Temperiergerätes weist die erste Temperiereinheit (7) wenigstens eine erste Ventilationseinrichtung (15) auf und die zweite Temperiereinheit (8) wenigstens eine zweite Ventilationseinrichtung.

Die Ventilationseinrichtung weist vorzugsweise einen, besonders bevorzugt jedoch mehrere, und zwar insbesondere zwei, Lüfter auf, welche auf dem Kühlkörper der ersten und der zweiten Temperiereinheit angeordnet sind. Die Lüfter sind vorzugsweise so geschaltet, dass die Lüfter Kühlungsluft auf die Kühlkörper der jeweiligen Temperiereinheit blasen.

Indem Kühlluft von einem jeweiliger Lüfter zu einem jeweiligen Kühlkörper befördert wird, ist die zu kühlende Fläche pro Lüfter reduziert und das pro Lüfter zu durchströmende Volumen entsprechend. Dies erlaubt weiter vorteilhaft, die Kühlleistung und damit den Stromverbrauch der Lüfter individuell zu regeln. Dies ist insbesondere bei einer Batteriebetriebenen mobilen Gerätevariante von Vorteil und führt außerdem zu einer verbesserten Genauigkeit der Temperaturregelung.

Der erste, bzw. der zweite Lüfter erzeugt im Betrieb des Temperiergerätes einen im Wesentlichen in axialer Richtung, d. h. einen senkrecht auf eine Bodenplatte des Kühlkörpers gerichteten Luftstrom. Die Bodenplatte des Kühlkörpers ist dabei unmittelbar flächig angrenzend an das Thermoelement und mit diesem thermisch gekoppelt. Die Kühlrippen des Kühlkörpers weisen dabei in axialer Richtung von der Bodenplatte weg zum Lüfter hin. Die einzelnen Kühlrippen sind dabei jeweils in zueinander parallelen, im Wesentlichen senkrecht zur Bodenplatte verlaufenden Ebenen angeordnet.

In einer bevorzugten Ausführungsform ist dabei wenigstens eine erste Ventilationseinrichtung (15) der ersten Temperiereinheit (7) den ersten Luftstrom erzeugt und die wenigstens eine zweite Ventilationseinrichtung (16) der zweiten Temperiereinheit (8) den zweiten Luftstrom erzeugt, wobei der erste Luftstrom der ersten Ventilationseinrichtung (15) in das Innere des Gehäuses (2) gerichtet ist und der zweite Luftstrom der zweiten Ventilationseinrichtung (16) in das Innere des Gehäuses (2) gerichtet ist.

Alternativ kann anstelle der Verwendung zweier getrennt arbeitender Ventilationseinrichtungen auch eine zentrale Ventilationseinrichtung vorgesehen sein. Die Ventilationseinrichtung kann in diesem Fall einen Luftstrom erzeugen, der nach der Ventilationseinrichtung in zwei unabhängige Luftströme aufgeteilt wird. Dies kann beispielsweise dadurch umgesetzt sein, dass der von der zentralen Ventilationseinrichtung erzeugte Luftstrom durch eine, als Strömungskanal geformte erste Röhre, kanalisiert und geführt wird. Nach einer bestimmten Länge teilt sich die erste Röhre in beispielsweise zwei getrennt verlaufende zweite und dritte Röhren auf, welche wiederum als Strömungskanäle ausgebildet sind. Dadurch wird der zentrale Luftstrom in zwei, insbesondere gleich starke einzelne Luftströme aufgespalten. Die einzelnen Luftströme werden durch die in dem Gehäuse des Temperiergerätes getrennt verlaufenden zweiten und dritten Strömungsröhren den Kühlkörpern so zugeführt, dass die zweite Röhre der ersten Temperiereinheit zukommt und die dritte Röhre der zweiten Temperiereinheit zukommt.

Die Aufteilung des zentralen Luftstromes kann in anteilig gleichem Verhältnis vorgenommen werden, so dass die zentrale Querschnittsfläche der zweiten und dritten Röhre jeweils die Hälfte der Querschnittsfläche der ersten Röhre aufweisen. Unterschiedlichste Verhältnisse der Querschnittsflächen zueinander sind denkbar. Ebenso können weitere Röhren umgesetzt werden, insbesondere können sich die zweite und dritte Röhre weiter verzweigen, so dass mehrere Luftströme aus unterschiedlichen Richtungen dem Kühlkörpern der ersten und dem Kühlkörper der zweiten Temperiereinheit zugeführt werden.

Die Querschnitte der Strömungsröhren können sich so ändern, dass sich diese zunehmend verjüngen, wodurch die Strömungsgeschwindigkeit des Luftstroms in der Röhre erhöht wird. Insbesondere kann das Ende der Röhren, aus welchen der Luftstrom in Richtung des Kühlkörpers austritt, verjüngt sein.

In einer bevorzugten Ausführugsform ist die wenigstens eine erste Ventilationseinrichtung der ersten Temperiereinheit zu wenigstens einer ersten Seitenwand des Gehäuses hin ausgerichtet und die wenigstens eine zweite Ventilationseinrichtung der zweiten Temperiereinheit zu der ersten Seitenwand des Gehäuses hin ausgerichtet, so dass durch wenigstens eine Öffnung in der ersten Seitenwand des Gehäuses der erste Luftstrom und oder der zweite Luftstrom in das Innere des Gehäuses gelangen, oder die wenigstens eine erste Ventilationseinrichtung der ersten Temperiereinheit zu wenigstens der ersten Seitenwand des Gehäuses hin ausgerichtet ist, so dass durch die wenigstens eine Öffnung in der ersten Seitenwand des Gehäuses der erste Luftstrom in das Innere des Gehäuses gelangt und die wenigstens eine zweite Ventilationseinrichtung der zweiten Temperiereinheit zu wenigstens einer zweiten Seitenwand des Gehäuses hin ausgerichtet ist, so dass durch wenigstens eine Öffnung in der zweiten Seitenwand des Gehäuses der zweite Luftstrom in das Innere des Gehäuses gelangt.

Besonders vorzugsweise ist der erste Lüfter so zu einer ersten Öffnung einer ersten Seitenwand oder eines ersten Wandabschnitts des Gehäuses ausgerichtet, dass der erste Lüfter die Luft durch diese erste Seitenwand oder diesen ersten Wandabschnitt ansaugt oder abgibt. Vorzugsweise ist der zweite Lüfter so zu einer zweiten Öffnung einer zweiten Seitenwand oder eines zweiten Wandabschnitts des Gehäuses ausgerichtet, dass der zweite Lüfter die Luft durch diese zweite Seitenwand oder diesen zweiten Wandabschnitt des Gehäuses ansaugt oder abgibt.

Somit können die Lüfter an benachbarten Seitenwänden und an die Öffnungen in den Seitenwänden so angrenzend angebracht sein, dass Luft zur Kühlung durch die Öffnungen im Betrieb des Lüfters an die Kühlkörper der jeweiligen Temperiereinheit direkt gefördert wird.

Damit ist es nicht notwendig konstruktiv aufwendige Lufteinlässe zu realisieren, die im Betrieb ausgehend von den Seitenwänden Luft zur Kühlung über Kanäle an die Lüfter leiten.

Alternativ können die Lüfter an einer gemeinsamen Öffnung in einer Seitenwand des Gehäuses nebeneinander angeordnet sein.

Erfindungsgemäß sind der wenigstens eine erste Kühlkörper und der wenigstens eine zweite Kühlkörper durch die Pumpeneinrichtung räumlich voneinander getrennt.

Dies ist insbesondere dann vorteilhaft, wenn die Lüfter an Luftöffnungen in gegenüberliegenden Seitenwänden des Gehäuses angebracht sind. Durch diese Anordnung kann der zwischen den Temperiereinheiten frei werdende Bauraum vorteilhaft genutzt werden.

Somit kann die Temperiereinrichtung kompakt ausgestaltet sein. Insbesondere kann die Temperiereinrichtung als Einschub - Modul ausgestaltet sein, so dass dieses als Ganzes aus dem Gehäuse entfernt und in dieses eingeschoben werden kann. Dies ermöglicht einen erleichterten Zugang zu der Fluidversorgungseinrichtung.

In einer besonders bevorzugten Ausführungsform weist die Fluidversorgungseinrichtung eine Fluidzuströmungseinrichtung auf, durch welche das Fluid von außen in das Gehäuse eingeführt wird.

Die Fluidzuströmungseinrichtung umfasst beispielsweise eine Leitung, welche beispielsweise in Form eines flexiblen Schlauchs ausgeführt ist und durch die das von außen zuströmende Fluid in das Gehäuse geleitet wird. Weiter umfasst die Fluidzuströmungseinrichtung ein Kupplungsstück, welches fest mit dem Gehäuse verbunden ist. Der Schlauch ist mit dem mechanischen Kupplungsstück fluidleitend verbunden und damit über das Kupplungsstück mechanisch an dem Gehäuse fixiert. Der Schlauch ist dabei aufsteckbar fluidleitend an das Kupplungsstück gekoppelt, so dass das Fluid durch das Kupplungsstück hindurch in das Innere des Gehäuses fließen kann.

Dabei ist die Fluidzuströmungseinrichtung vorzugsweise zur Einführung wenigstens eines Teils des durch die Fluidabströmungseinrichtung aus dem Gehäuse herausgeführten Fluids in das Gehäuse vorgesehen, so dass sich ein wenigstens teilweiser Fluidkreislauf ergibt.

Dabei weist das durch die Fluidzuströmungseinrichtung in das Gehäuse eingeführte Fluid eine andere, vorzugsweise geringere, weiter vorzugsweise höhere, Temperatur und/oder einen anderen, vorzugsweise geringeren, Druck auf, als das durch die Fluidabströmungseinrichtung aus dem Gehäuse herausgeführte Fluid.

In einer weiteren bevorzugten Ausführungsform der Erfindung weist die Fluidversorgungseinrichtung einen im Gehäuse angeordneten Fluidbehälter auf. Somit kann der Benutzer das Gerät auf bequeme Weise bei Inbetriebnahme mit Fluid befüllen.

In einer weiteren bevorzugten Ausführungsform der Erfindung ist die Fluidzuströmungseinrichtung mit dem Fluidbehälter fluidleitend verbunden. Auf diese Weise kann der Fluidbehälter in den Fluidkreislauf eingebunden werden und dabei als Puffer für die zirkulierende Fluidmenge dienen, wobei eine beispielsweise durch Leckagen oder Verdunstung verloren gehende Fluidmenge automatisch ausgeglichen wird.

In einer weiteren bevorzugten Ausführungsform der Erfindung sind die erste Temperiereinheit und die zweite Temperiereinheit im Wesentlichen gleich aufgebaut.

Dies vereinfacht die Konstruktion des Temperiergerätes sowie die Logistik und damit die Kosten bei dessen Herstellung.

Die Erfindung betrifft auch ein Verfahren zur Herstellung eines medizinischen Temperiergeräts, insbesondere eines Temperiergeräts gemäß einem der Patentansprüche, aufweisend wenigstens einen der folgenden, insbesondere jeden der folgenden Schritte:
- Bereitstellen und Anordnen wenigstens eines Gehäuses (2),
- optional: Bereitstellen und Anordnen wenigstens einer Fluidversorgungseinrichtung (3), durch welche ein Fluid in dem Gehäuse (2) bereitgestellt wird,
- Bereitstellen und Anordnen wenigstens einer im Gehäuse (2) angeordneten Temperiereinrichtung (4), die das durch das Gehäuse (2) strömende Fluid temperiert,
- optional: Bereitstellen und Anordnen wenigstens einer Fluidabströmungseinrichtung (5), durch welche das Fluid mit einer im Wesentlichen vorbestimmten Temperatur aus dem Gehäuse (2) herausgeführt wird,
- optional: Bereitstellen und Anordnen wenigstens einer im Gehäuse (2) angeordneten Pumpeneinrichtung (6), welche das durch das Gehäuse (2) strömende Fluid mit Druck beaufschlagt, und

wobei diese wenigstens eine Temperiereinrichtung (4) wenigstens eine erste Temperiereinheit (7) und wenigstens eine zweite Temperiereinheit (8) aufweist,
wobei die erste Temperiereinheit (7) wenigstens ein erstes thermoelektrisches Element (9), insbesondere wenigstens ein Peltier-Element, wenigstens ein von Fluid durchströmbares erstes Wärmetauscherelement (10) zur Übertragung von Wärme auf das oder von dem Fluid und wenigstens einen ersten Kühlkörper (11) aufweist, und
wobei die zweite Temperiereinheit (8) wenigstens ein zweites thermoelektrisches Element (12), insbesondere wenigstens ein Peltier-Element, wenigstens ein von Fluid durchströmbares zweites Wärmetauscherelement (13) zur Übertragung von Wärme auf das oder von dem Fluid und wenigstens einen zweiten Kühlkörper (14) aufweist, und
das medizinische Temperiergerät (1) eingerichtet ist wenigstens einen Luftstrom zu erzeugen,
   - wobei das medizinische Temperiergerät (1) so eingerichtet wird bzw. ist, dass ein erster Luftstrom auf den wenigstens einen ersten Kühlkörper (11) der ersten Temperiereinheit (7) gerichtet ist und ein zweiter Luftstrom auf den wenigstens einen zweiten Kühlkörper (14) der zweiten Temperatureinheit (8) gerichtet ist,
wobei der wenigstens eine erste Kühlkörper und der wenigstens eine zweite Kühlkörper durch die Pumpeneinrichtung räumlich voneinander getrennt sind.

Weitere bevorzugte Ausgestaltungsmerkmale des erfindungsgemäßen Verfahrens zur Herstellung des Temperiergeräts ergeben sich aus den zuvor beschriebenen optionalen und bevorzugten Ausgestaltungen des erfindungsgemäßen Temperiergeräts und aus der nachfolgenden Beschreibung der Figuren. Weitere bevorzugte Ausgestaltungsmerkmale des erfindungsgemäßen Temperiergeräts ergeben sich aus der nachfolgenden Beschreibung der Figuren.

Die Erfindung wird im Folgenden anhand eines Ausführungsbeispiels unter Bezugnahme auf die beigefügten Zeichnungen erläutert. Dabei zeigen:
Fig. 1: Perspektivische Ansicht eines erfindungsgemäßen Temperiergerätes 1.
Fig. 2: Perspektivische Draufsicht der Temperiereinrichtung 4 eines beispielhaften erfindungsgemäßen medizinischen Temperiergerätes 1.
Fig. 3: Schematische Schnittansicht durch die Rückwand des beispielhaften medizinischen Temperiergerätes 1.

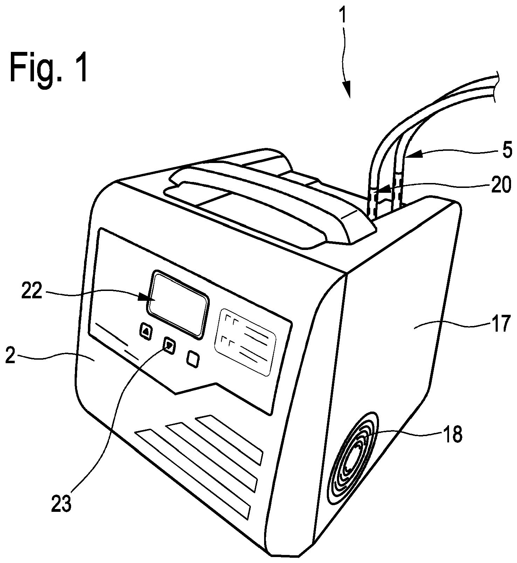

Fig.1 zeigt das erfindungsgemäße Temperiergerät 1 mit einem Gehäuse 2, welches eine Seitenwand 17 umfasst, die eine Öffnung 18 aufweist, durch welche Luft in das Innere des Gehäuses 2 einströmen kann, mit einer Fluidzuströmungseinrichtung 20, welche aus einer als Schlauch ausgebildeten flexiblen Leitung besteht oder diese umfasst, und mit dem Kupplungsstück fluidleitend verbunden ist, mit einer Fluidabströmungseinrichtung 5, welche aus einer als Schlauch ausgebildeten flexiblen Leitung besteht oder diese umfasst, und mit dem Kupplungsstück fluidleitend verbunden ist, mit einem Bediendisplay 22, welches dem Bediener ermöglicht eine Temperatur des Fluids für eine Behandlung vorzuwählen, so dass die vorgewählte Temperatur durch eine Temperaturregelung mittels der Temperiereinrichtung 4 über den Zeitraum der Behandlung im Wesentlichen konstant gehalten wird. Dabei kann der Bediener mittels Bedienelement 23, welche in der vorliegenden beispielhaften Ausführungsform als Druckknöpfe ausgebildet sind, die gewünschte Temperatur anwählen.

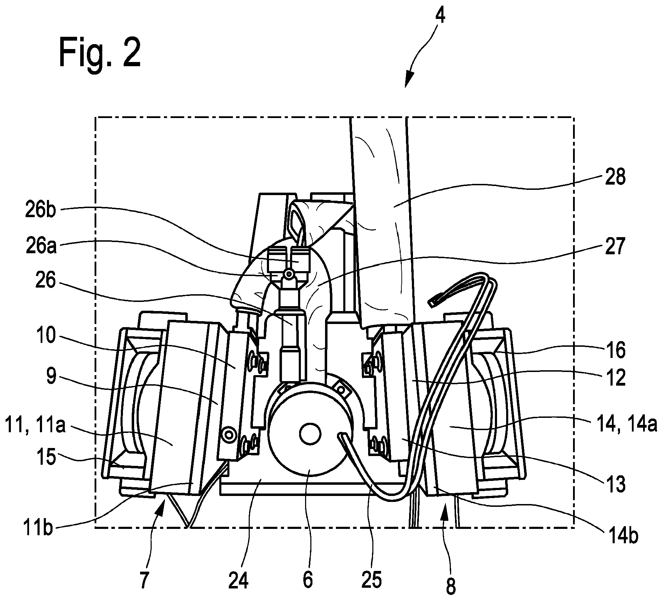

In Fig. 2 ist eine perspektivische Draufsicht der Temperiereinrichtung 4 eines beispielhaften erfindungsgemäßen medizinischen Temperiergerätes 1 gezeigt. Dabei sind die erste Temperiereinheit 7 und die zweite Temperiereinheit 8 als räumlich getrennt voneinander und als spiegelsymmetrisch zueinander angeordnet dargestellt. Zwischen der ersten Temperiereinheit 7 und der zweiten Temperiereinheit 8 befindet sich eine Kunststoffschaumform 24, wobei die erste und die zweite Temperiereinheit 7, 8 randseitig an der Kunststoffschaumform 24 angeordnet und mechanisch daran befestigt sind. Die erste Temperiereinheit 7 ist dabei an der Kunstoffschaumform 24 hochkannt befestigt. Damit ist gemeint, dass die aneinander zum Wärmetransport angrenzenden, parallel verlaufenden Flächen zwischen dem ersten Wärmetauscherelement 10 und dem ersten thermoelektrischen Element 9 im Wesentlichen senkrecht zur Darstellungsebene stehen. Die zweite Temperiereinheit 8 ist ebenso an der Kunstoffschaumform 24 hochkannt befestigt. Mittig, zwischen der hochkannt angeordneten ersten Temperiereinheit 7 und der hockannt angeordneten zweiten Temperiereinheit 8 ist die Pumpeneinrichtung 6 gezeigt. Die Pumpeneinrichtung 6 besteht oder umfasst dabei eine Pumpe 6, eine elektrische Zuleitung 25 und einer Steuereinheit, nicht gezeigt. Die Pumpe 6 ist dabei an der Kunststoffschaumform 24 mechanisch befestigt, so dass die Pumpe 6 in ihrer Position zwischen den Temperiereinheiten 7, 8 mittels der Kunststoffschaumform 24 mechanisch fixiert wird. Ausgehend von der Pumpe verläuft eine erste Fluidleitung 26, welche in einem T-Stück endet. Dabei dient der erste Teilabschnitt des T-Stücks 26a der Fluidleitung 26 zum Belüften des Fluidkreislaufs. Das Belüften kann beispielsweise über ein Belüftungsventil, welches nicht in Fig. 2 gezeigt ist erfolgen. Eine Belüftung ist notwendig, um eventuell vorhandene Lufteinschlüsse im Fluidkreislauf zu beseitigen. An den zweiten Teilabschnitt des T-Stücks 26b der Fluidleitung 26 ist eine weitere Fluidleitung 27 angeschlossen, welche die Pumpe 6 mit dem zweiten Wärmetauscherelement 13 der zweiten Temperiereinheit 8 fluidleitend verbindet. Das durch die Pumpe 6 geförderte Fluid strömt durch das zweite Wärmetauscherelement 13. Wird vom Benutzer eine Temperatur über den Bedienelement 23 ausgewählt, die niederiger als diejenige des Fluids ist, so wird das Fluid gekühlt. Dabei gibt das die Temperiereinheit 8 durchströmenden Fluid seine Wärmeenergie an das Wärmetauscherelement 13 ab. Im Falle der Erwärmung des Fluids nimmt das Fluid Wärmeenergie vom Wärmetauscherelement 13 beim durchströmen des Wärmetauscherelementes 13 von diesem auf. Das Fluid strömt von dem zweiten Wärmetauscherelement 13 über die Fluidleitung 28 zur Fluidabströmungseinrichtung 5, von wo aus es aus dem Gehäuse heraus in Richtung der zu behandelnden Körperfläche gelangt. Dort nimmt das Fluid über eine Behandlungsmanchette, beispielsweise Wärme von der zu behandelenden Körperfläche auf. Das erwärmte Fluid strömt über die Fluidzuströmungseinrichtung 20 zurück in das Temperiergerät 1 und gelangt über die Fluidversorgungseinrichtung 3 in das Wärmetauscherelement 10 der ersten Temperiereinheit 7 und von dort wiederum zur Pumpe 6, so dass sich ein Fluidkreislauf ergibt.

Die Lüfter 14, 15 sind jeweils als herkömmlicher Lüfter 14, 15 mit einem Turbinenrad ausgeführt. Es sind jedoch auch andere Bauformen für die Lüfter möglich. Die Lüfter 14, 15 erzeugen im Betrieb des Temperiergerätes 1 einen im Wesentlichen in axialer Richtung, d. h. einen senkrecht auf die Bodenplatte 11a, 14b der Kühlkörper 11, 14 gerichteten Luftstrom.

Die erste Temperiereinheit 7 und die zweite Temperiereinheit 8 sind als sogenannte "Liquid-to-Air-Module" ausgeführt. In dem in Fig. 2 gezeigten Ausführungsbeispiel ist dabei der Aufbau der ersten Temperiereinheiten 7 an sich mit dem Aufbau der zweiten Temperiereinheit 8 im Wesentlichen gleich. Die erste Temperiereinheit 7 weist ein Peltier-Element 9 auf, welches zwischen dem Kühlkörper 11 und dem Wärmetauscherelement 10 angeordnet ist. Dabei grenzen die Bodenplatte des Kühlkörpers 11a und das Wärmetauscherelement 10 unmittelbar und großflächig aneinander, so dass sich eine große gemeinsame Berührungsfläche zwischen Kühlkörper 11 und Wärmetauscherlement 10 ergibt, damit sich zischen dem Kühlkörper 11 und dem Wärmetauscherelement 10 eine effiziente Wärmeübertragung durch einen gleichmäßigen Wärmestrom über die Berührungsfläche ausbildet. Der ersten Temperiereinheit 7 entsprechend weist die zweite Temperiereinheit 8 ein Peltier-Element 12 auf, welches zwischen der Bodenplatte des Kühlkörpers 14b und dem Wärmetauscherelement 13 angeordnet ist. Der Kühlkörper 11 der ersten Temperiereinheit 7 und der Kühlkörper 14 der zweiten Temperiereinheit 8 weist eine Vielzahl (im Ausführungsbeispiel etwa 50) von rechteckigen, parallel zueinander angeordneten und voneinander beabstandeten Rippen 11a, 14a auf. Die Rippen 11a, stehen senkrecht auf einer Bodenplatte 11b des Kühlkörpers 11 der ersten Temperiereinheit 7 und die Rippen 14b stehen senkrecht auf einer Bodenplatte 14b des Kühlkörpers 14 der zweiten Temperiereinheit 8. Die Kühlkörper 11, 14 sind vorzugsweise im sogenannten "Skived-Fin"-Verfahren hergestellt.

Die Wärmetauscherelemente 10, 13 bestehen im Wesentlichen aus einem vorzugsweise weitgehend oder vollständig massiven Metallblock auf. Durch den Metallblock des ersten Wärmetauscherelementes 10 und den Metallblock der das zweite Wärmetauscherelementes 13 formt führt eine (in den Figuren nicht sichtbare) interne Fluidleitung für das zu temperierende Fluid führt. Die interne Fluidleitung kann insbesondere in Schlangenlinien oder spiralförmig sowie in einer oder in mehreren Ebenen durch den Metallblock verlaufen.

Für eine medizinische Wärme - oder Kältebehandlung ist es wünschenswert, die Temperatur, mit der das Fluid das Temperiergerät verlässt, möglichst konstant zu halten, bei einer Kältebehandlung beispielsweise auf 12 °C. Die hierfür notwendige Temperaturregelung kann auf einfache Weise dadurch vorgenommen werden, dass ein (nicht dargestellter) Temperatursensor die Temperatur des abströmenden Fluids misst und dass ein oder beide Peltier - Elemente 9, 12 ein- bzw. ausgeschaltet werden, sobald die gemessene Temperatur je nach Anwendungsfall unter oder über der Solltemperatur liegt. Das Ein - bzw. Ausschalten der Peltier - Elemente 9, 12 kann beispielsweise mittels einer elektronischen Steuereinrichtung (in den Figuren nicht sichtbar) erfolgen, welche im Gehäuse des Temperiergerätes 1 untergebracht ist. Die elektronische Steuereinrichtung kann auch durch die Bedienung des Gerätes mittels Bedienelement 23 und Bediendisplay 22 gesteuert werden.

Dabei ist vorteilhafter Weise der Fluidkreislauf so ausgestaltet, dass die Pumpeneinrichtung 16 fluidleitend zwischen die beiden Wärmetauscher 8, 9 und mit diesen in Reihe geschaltet ist, so dass eine Erwärmung des gepumpten Fluids durch die Abwärme der Pumpeneinrichtung 16 von dem darauf folgenden Wärmetauscher 8, 9 mittels dessen Kühlregelung wieder ausgeglichen wird.

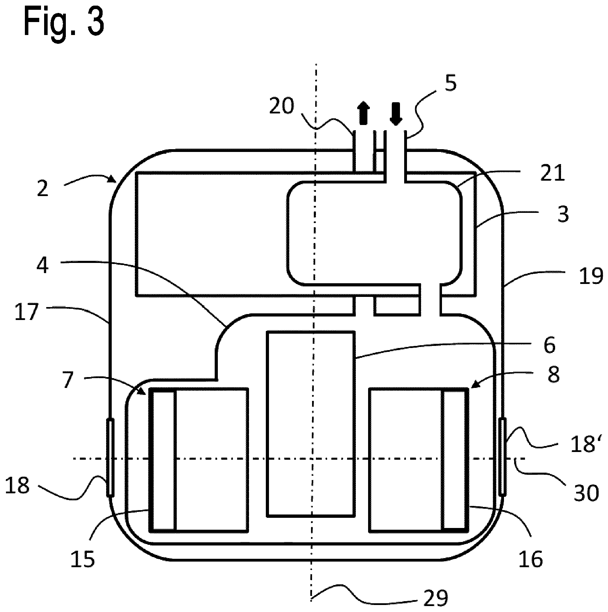

In Fig. 3 ist eine schematische Schnittansicht durch die Rückwand des beispielhaften medizinischen Temperiergerätes 1 dargestellt. Im oberen Bereich des Gehäuses 2 ist die Fluidversorgungseinrichtung 3 dargestellt, welche einen Fluidbehälter 21 umfasst, oder aus diesem besteht. Der Fluidbehälter 21 ist in fluidleitender Verbindung mit Fluidzuströmungseinrichtung 20 durch welche das von der Behandlungsmanchette (nicht in der Figur sichtbar) kommende Fluid in das Gehäuse 2 hinein befördert wird. Der Fluidbehälter 21 ist auch in fluidleitender Verbindung mit der Temperiereinrichtung 4. Zur Fluidzuströmungseinrichtung 20 benachbart dargestellt ist die Fluidabströmungseinrichtung 5, durch welche das Fluid von der Temperiereinrichtung 4 ausgehend aus dem Gehäuse 2 heraus und in Richtung der Behandlungsmanchette transportiert wird. Die Strömungsrichtungen des Fluids sind durch Pfeile an den Zu - bzw. Abströmungseinrichtungen 5, 20 gekennzeichnet.

Die erste Temperiereinheit 7 und die zweite Temperiereinheit 8 sind symmetrisch im Gehäuse 2 zu der Symmetrieachse 29 angeordnet. Zwischen der ersten Temperiereinheit 7 und der zweiten Temperiereinheit 8 ist die Pumpe 6 symmetrisch zur Symmetrieachse 29 angeordnet. Der Aufbau der Temperiereinheit 7 und der Aufbau der Temperiereinheit 8 ist dabei im Wesentlichen gleich, erscheint jedoch aufgrund der symmetrischen Anordnung der Temperiereinheiten 7, 8 zueinander spiegelsymmetrisch. Das Gehäuse 2 weist eine erste Seitenwand 17 mit einer ersten Öffnung 18 auf, sowie eine zweite Seitenwand 19 mit einer Öffnung 18`. Die Öffnungen 18, 18' sind so in den Seitenwänden 17, 19 positioniert, dass entlang einer Ventilationsachse 30 Luft zum Kühlen der ersten Temperiereinheit 7 durch die Öffnung 18 in das Gehäuse hinein strömen kann und Luft durch die Öffnung 18' zum Kühlen der zweiten Temperiereinheit 8 in das Gehäuse 2 hinein strömen kann. Die Luft wird dabei durch den Lüfter 15 der ersten Temperiereinheit 7 und den Lüfter 16 der zweiten Temperiereinheit 8 angesaugt, so dass die Luft im Wesentlichen entlang der Ventilationsachse 30 in das Gehäuse strömt.

### Bezugszeichen

- 1: Temperiergerät
- 2: Gehäuse
- 3: Fluidversorgungseinrichtung
- 4: Temperiereinrichtung
- 5: Fluidabströmungseinrichtung
- 6: Pumpeneinrichtung
- 7: Erste Temperiereinheit
- 8: Zweite Temperiereinheit
- 9: Erstes thermoelektrisches Element
- 10: Erstes Wärmetauscherelement
- 11: Erster Kühlkörper
- 11a: Kühlrippen des ersten Kühlkörpers
- 11b: Bodenplatte des ersten Kühlkörpers
- 12: Zweiter thermoelektrisches Element
- 13: Zweites Wärmetauscherelement
- 14: Zweiter Kühlkörper
- 14a: Kühlrippen des zweiten Kühlkörpers
- 14b: Bodenplatte des zweiten Kühlkörpers
- 15: Erste Ventilationseinrichtung
- 16: Zweite Ventilationseinrichtung
- 17: Erste Seitenwand
- 18: Öffnung
- 18': Öffnung
- 19: Zweite Seitenwand
- 20: Fluidzuströmungseinrichtung
- 21: Fluidbehälter
- 22: Bediendisplay
- 23: Bedienelement
- 24: Kunststoffschaumform
- 25: Elektrische Zuleitung
- 26: Erste Fluidleitung
- 26a: Erste Teilabschnitt T-Stück
- 26b: Zweite Teilabschnitt T-Stück
- 27: Zweite Fluidleitung
- 28: Dritte Fluidleitung
- 29: Symmetrieachse
- 30: Ventilationsachse

## Patentansprüche

1. Medizinisches Temperiergerät (1) mit,
wenigstens einem Gehäuse (2),
wenigstens einer Fluidversorgungseinrichtung (3), durch welche ein Fluid in dem Gehäuse (2) bereitgestellt wird,
wenigstens einer im Gehäuse (2) angeordneten Temperiereinrichtung (4), die das durch das Gehäuse (2) strömende Fluid temperiert,
wenigstens einer Fluidabströmungseinrichtung (5), durch welche das Fluid mit einer im Wesentlichen vorbestimmten Temperatur aus dem Gehäuse (2) herausgeführt wird,
wenigstens einer im Gehäuse (2) angeordneten Pumpeneinrichtung (6), welche das durch das Gehäuse (2) strömende Fluid mit Druck beaufschlagt, und
dass diese wenigstens eine Temperiereinrichtung (4) wenigstens eine erste Temperiereinheit (7) und wenigstens eine zweite Temperiereinheit (8) aufweist,
wobei die erste Temperiereinheit (7) wenigstens ein erstes thermoelektrisches Element (9), insbesondere wenigstens ein Peltier-Element, wenigstens ein von Fluid durchströmbares erstes Wärmetauscherelement (10) zur Übertragung von Wärme auf das oder von dem Fluid und wenigstens einen ersten Kühlkörper (11) aufweist, und
wobei die zweite Temperiereinheit (8) wenigstens ein zweites thermoelektrisches Element (12), insbesondere wenigstens ein Peltier-Element, wenigstens ein von Fluid durchströmbares zweites Wärmetauscherelement (13) zur Übertragung von Wärme auf das oder von dem Fluid und wenigstens einen zweiten Kühlkörper (14) aufweist, und
das medizinische Temperiergerät (1) eingerichtet ist wenigestens einen Luftstrom zu erzeugen,
wobei
ein erster Luftstrom auf den wenigstens einen ersten Kühlkörper (11) der ersten Temperiereinheit (7) gerichtet ist und ein zweiter Luftstrom auf den wenigstens einen zweiten Kühlkörper (14) der zweiten Temperatureinheit (8) gerichtet ist,
**dadurch gekennzeichnet,**
**dass** der wenigstens eine erste Kühlkörper (11) und der wenigstens eine zweite Kühlkörper (14) durch die Pumpeneinrichtung (6) räumlich voneinander getrennt sind.

2. Medizinisches Temperiergerät gemäß Anspruch 1, wobei der
erste Kühlkörper (11) der ersten Temperiereinheit (7) einen Bereich mit Kühlrippen (11a) aufweist und der Bereich mit Kühlrippen (11a) der ersten Temperiereinheit (7) von dem ersten Luftstrom durchströmt wird, und
der zweite Kühlkörper (14) der zweiten Temperiereinheit (8) einen Bereich mit Kühlrippen (14a) aufweist und der Bereich mit Kühlrippen (14a) der zweiten Temperiereinheit (8) von dem zweiten Luftstrom durchströmt wird.

3. Medizinisches Temperiergerät gemäß Anspruch 2, wobei der erste Luftstrom im Wesentlichen parallel zu den Ebenen verläuft, in denen sich die jeweiligen Kühlrippen (11a) des ersten Kühlkörpers (11) der ersten Temperiereinheit (7) erstrecken und welche senkrecht zu der Ebene angeordnet sind, die sich durch das unmittelbar flächige aneinandergrenzen von dem ersten Thermoelement (12) und dem ersten Kühlkörper (11) ergibt und entsprechend verläuft der zweite Luftstrom im Wesentlichen parallel zu den Ebenen, in denen sich die jeweiligen Kühlrippen (14a) des zweite Kühlkörpers (14) der zweiten Temperiereinheit (8) erstrecken und welche senkrecht zu der Ebene angeordnet sind, die sich durch das unmittelbar flächige aneinandergrenzen von dem zweitem Thermoelement (12) und dem zweiten Kühlkörper (14) ergibt

4. Medizinisches Temperiergerät gemäß Anspruch 2, wobei die Kühlrippen (11a) des ersten Kühlkörpers (11) der ersten Temperiereinheit (7) und/oder die Kühlrippen (14a) des zweiten Kühlkörpers (14) der zweiten Temperiereinheit (8) im "Skived-Fin"-Verfahren hergestellt sind.

5. Medizinisches Temperiergerät gemäß einem der vorhergehenden Ansprüche, wobei die Kühlkörper (11, 14) der ersten und der zweiten Temperiereinheit (7, 8) aus Kupfer gefertigt sind.

6. Medizinisches Temperiergerät gemäß einem der vorhergehenden Ansprüche, wobei die erste Temperiereinheit (7) und die zweite Temperiereinheit (8) im Wesentlichen spiegelsymmetrisch zueinander angeordnet sind.

7. Medizinisches Temperiergerät gemäß einem der vorhergehenden Ansprüche, wobei die erste Temperiereinheit (7) wenigstens eine erste Ventilationseinrichtung (15) aufweist und die zweite Temperiereinheit (8) wenigstens eine zweite Ventilationseinrichtung (16) aufweist.

8. Medizinisches Temperiergerät gemäß Anspruch 7, wobei die wenigstens eine erste Ventilationseinrichtung (15) der ersten Temperiereinheit (7) den ersten Luftstrom erzeugt und die wenigstens eine zweite Ventilationseinrichtung (16) der zweiten Temperiereinheit (8) den zweiten Luftstrom erzeugt, wobei der erste Luftstrom der ersten Ventilationseinrichtung (15) in das Innere des Gehäuses (2) gerichtet ist und der zweite Luftstrom der zweiten Ventilationseinrichtung (16) in das Innere des Gehäuses (2) gerichtet ist.

9. Medizinisches Temperiergerät gemäß Anspruch 8, wobei die
wenigstens eine erste Ventilationseinrichtung (15) der ersten Temperiereinheit (7) zu wenigstens einer ersten Seitenwand (17) des Gehäuses (2) hin ausgerichtet ist und die wenigstens eine zweite Ventilationseinrichtung (16) der zweiten Temperiereinheit (8) zu der ersten Seitenwand (17) des Gehäuses (2) hin ausgerichtet ist, so dass durch wenigstens eine Öffnung (18) in der ersten Seitenwand (17) des Gehäuses (2) der erste Luftstrom und oder der zweite Luftstrom in das Innere des Gehäuses (2) gelangen, oder
die wenigstens eine erste Ventilationseinrichtung (15) der ersten Temperiereinheit (7) zu wenigstens der ersten Seitenwand (17) des Gehäuses (2) hin ausgerichtet ist, so dass durch die wenigstens eine Öffnung (18) in der ersten Seitenwand (17) des Gehäuses (2) der erste Luftstrom in das Innere des Gehäuses (2) gelangt und die wenigstens eine zweite Ventilationseinrichtung (16) der zweiten Temperiereinheit (8) zu wenigstens einer zweiten Seitenwand (19) des Gehäuses (2) hin ausgerichtet ist, so dass durch wenigstens eine Öffnung (18') in der zweiten Seitenwand (19) des Gehäuses (2) der zweite Luftstrom in das Innere des Gehäuses (2) gelangt.

10. Medizinisches Temperiergerät gemäß einem der vorherigen Ansprüche, wobei die Fluidversorgungseinrichtung (3) eine Fluidzuströmungseinrichtung (5) aufweist, durch welche das Fluid von außen in das Gehäuse (2) eingeführt wird.

11. Medizinisches Temperiergerät gemäß Anspruch 10, wobei die Fluidzuströmungseinrichtung (5) zur Einführung wenigstens eines Teils des durch die Fluidabströmungseinrichtung (20) aus dem Gehäuse (2) herausgeführten Fluids in das Gehäuse (2) vorgesehen ist, so dass sich ein wenigstens teilweiser Fluidkreislauf ergibt, wobei das durch die Fluidzuströmungseinrichtung (5) in das Gehäuse (2) eingeführte Fluid eine andere, vorzugsweise geringere, weiter vorzugsweise höhere, Temperatur und/oder einen anderen, vorzugsweise geringeren, Druck aufweist als das durch die Fluidabströmungseinrichtung (20) aus dem Gehäuse (2) herausgeführte Fluid.

12. Medizinisches Temperiergerät gemäß einem der vorhergehenden Ansprüche, wobei die Fluidversorgungseinrichtung (3) einen im Gehäuse (1) angeordneten Fluidbehälter (21) aufweist.

13. Medizinisches Temperiergerät gemäß den Ansprüchen 10 und 12, wobei die Fluidzuströmungseinrichtung (5) mit dem Fluidbehälter (21) fluidleitend verbunden ist.

14. Verfahren zur Herstellung eines medizinischen Temperiergeräts, insbesondere eines Temperiergeräts gemäß einem der vorangehenden Patentansprüche, aufweisend die Schritte:
• Bereitstellen und Anordnen wenigstens eines Gehäuses (2),
• Bereitstellen und Anordnen wenigstens einer im Gehäuse (2) angeordneten Temperiereinrichtung (4), die das durch das Gehäuse (2) strömende Fluid temperiert,
wobei diese wenigstens eine Temperiereinrichtung (4) wenigstens eine erste Temperiereinheit (7) und wenigstens eine zweite Temperiereinheit (8) aufweist, wobei die erste Temperiereinheit (7) wenigstens ein erstes thermoelektrisches Element (9), insbesondere wenigstens ein Peltier-Element, wenigstens ein von Fluid durchströmbares erstes Wärmetauscherelement (10) zur Übertragung von Wärme auf das oder von dem Fluid und wenigstens einen ersten Kühlkörper (11) aufweist, und
wobei die zweite Temperiereinheit (8) wenigstens ein zweites thermoelektrisches Element (12), insbesondere wenigstens ein Peltier-Element, wenigstens ein von Fluid durchströmbares zweites Wärmetauscherelement (13) zur Übertragung von Wärme auf das oder von dem Fluid und wenigstens einen zweiten Kühlkörper (14) aufweist, und das medizinische Temperiergerät (1) eingerichtet ist wenigstens einen Luftstrom zu erzeugen,
• wobei das medizinische Temperiergerät (1) so eingerichtet wird, dass ein erster Luftstrom auf den wenigstens einen ersten Kühlkörper (11) der ersten Temperiereinheit (7) gerichtet ist und ein zweiter Luftstrom auf den wenigstens einen zweiten Kühlkörper (14) der zweiten Temperiereinheit (8) gerichtet ist,
**dadurch gekennzeichnet,**
**dass** der wenigstens eine erste Kühlkörper (11) und der wenigstens eine zweite Kühlkörper (14) durch die Pumpeneinrichtung (6) räumlich voneinander getrennt sind.

## Claims

1. Medical temperature control apparatus (1)with,
at least one housing (2),
at least one fluid supply device (3), by means of which a fluid is provided in the housing (2),
at least one temperature control device (4) arranged in the housing (2), which controls the temperature of the fluid flowing through the housing (2),
at least one fluid inflow device (5), through which the fluid is led out of the housing (2) at a substantially predetermined temperature,
at least one pump device (6) arranged in the housing (2), which pressurizes the fluid flowing through the housing (2), and
in that this at least one temperature control device (4) comprises at least one first temperature control unit (7) and at least one second temperature control unit (8), the first temperature control unit (7) having at least one first thermoelectric element (9), in particular at least one Peltier element, at least one first heat exchanger element (10), through which fluid can flow, for transferring heat to or from the fluid and at least one first heat sink (11), and the second temperature control unit (8) comprising at least one second thermoelectric element (12), in particular at least one Peltier element, and the second temperature control unit (8) comprising at least one second thermoelectric element (12), in particular at least one Peltier element, for transferring heat to or from the fluid, in particular at least one Peltier element, at least one second heat exchanger element (13), through which fluid can flow, for transferring heat to or from the fluid and at least one second heat sink (14), and the medical temperature control apparatus (1) is configured to generate at least one air flow, a first air flow being directed towards the at least one first heat sink (11) of the first temperature control unit (7) and a second air flow being directed towards the at least one second heat sink (14) of the second temperature control unit (8),
**characterized in that**
the at least one first heat sink (11) and the at least one second heat sink (14) are spatially separated from one another by the pump device (6).

2. Medical temperature control apparatus accordingto claim 1, wherein the first heat sink (11) of the first temperature control unit (7) comprises a region with cooling fins (11a) and the region with cooling fins (11a) of the first temperature control unit (7) is flowed through by the first air flow, and the second heat sink (14) of the second temperature control unit (8) comprises a region with cooling fins (14a) and the region with cooling fins (14a) of the second temperature control unit (8) is flowed through by the second air flow.

3. Medical temperature control apparatus according to claim 2, wherein the first air flow runs substantially parallel to the planes in which the respective cooling fins (11a) of the first heat sink (11) of the first temperature control unit (7) extend and which are arranged perpendicular to the plane which results from the direct planar adjacency of the first thermocouple (12) and the first heat sink (11), and correspondingly the second air flow runs substantially parallel to the planes in which the respective cooling fins (11a) of the first heat sink (11) extend, in which the respective cooling fins (14a) of the second heat sink (14) of the second temperature control unit (8) extend and which are arranged perpendicularly to the plane resulting from the direct planar adjacency of the second thermocouple (12) and the second heat sink (14).

4. Medical temperature control apparatus according to claim 2, wherein the cooling fins (11a) of the first heat sink (11) of the first temperature control unit (7) and/or the cooling fins (14a) of the second heat sink (14) of the second temperature control unit (8) are produced by the "skived fin" method.

5. Medical temperature control apparatus according to one of the preceding claims, wherein the heat sinks (11, 14) of the first and second temperature control units (7, 8) are made of copper.

6. Medical temperature control apparatus according to any one of the preceding claims, wherein the first temperature control unit (7) and the second temperature control unit (8) are arranged substantially mirror-symmetrically to each other.

7. Medical temperature control apparatus according to one of the preceding claims, wherein the first temperature control unit (7) comprises at least one first ventilation device (15) and the second temperature control unit (8) comprises at least one second ventilation device (16).

8. Medical temperature control apparatus according to claim 7, wherein the at least one first ventilation device (15) of the first temperature control unit (7) generates the first air flow and the at least one second ventilation device (16) of the second temperature control unit (8) generates the second air flow, wherein the first air flow of the first ventilation device (15) is directed into the interior of the housing (2) and the second air flow of the second ventilation device (16) is directed into the interior of the housing (2).

9. Medical temperature control apparatus according to claim 8, wherein the at least one first ventilation device (15) of the first temperature control unit (7) is directed towards at least one first side wall (17) of the housing (2) and the at least one second ventilation device (16) of the second temperature control unit (8) is directed towards the first side wall (17) of the housing (2), so that the first air flow and or the second air flow enter the interior of the housing (2) through at least one opening (18) in the first side wall (17) of the housing (2), or the at least one first ventilation device (15) of the first temperature control unit (7) is directed towards at least the first side wall (17) of the housing (2), so that the first air flow passes through the at least one opening (18) in the first side wall (17) of the housing (2) into the interior of the housing (2) and the at least one second ventilation device (16) of the second temperature control unit (8) is directed towards at least one second side wall (19) of the housing (2), so that the second airflow passes through at least one opening (18') in the second side wall (19) of the housing (2) into the interior of the housing (2).

10. Medical temperature control apparatus according to one of the preceding claims, wherein the fluid supply device (3) comprises a fluid inflow device (5) through which the fluid is introduced into the housing (2) from the outside.

11. Medical temperature control apparatus according to claim 10, wherein the fluid inflow device (5) is provided for introducing at least a part of the fluid led out of the housing (2) through the fluid discharge device (20) into the housing (2), so that an at least partial fluid circulation results, wherein the fluid introduced into the housing (2) through the fluid inflow device (5) has a different, preferably lower, further preferably higher, temperature and/or a different, preferably lower, pressure than the fluid led out of the housing (2) through the fluid discharge device (20).

12. Medical temperature control apparatus according to one of the preceding claims, wherein the fluid supply device (3) comprises a fluid container (21) arranged in the housing (1).

13. Medical temperature control apparatus according to claims 10 and 12, wherein the fluid inflow device (5) is connected to the fluid container (21) in a fluid-conducting manner.

14. Method for manufacturing a medical temperature control apparatus, in particular a temperature control apparatus according to one of the preceding claims, comprising the steps of
- Providing and arranging at least one housing (2),
- Providing and arranging at least one temperature control device (4) which is arranged in the housing (2) and which regulates the temperature of the fluid flowing through the housing (2),
wherein this at least one temperature control device (4) comprises at least one first temperature control unit (7) and at least one second temperature control unit (8), wherein the first temperature control unit (7) has at least one first thermoelectric element (9), in particular at least one Peltier element, at least one first heat exchanger element (10) through which fluid can flow for transferring heat to or from the fluid, and at least one first heat sink (11), and wherein the second temperature control unit (8) comprises at least one second thermoelectric element (12), in particular at least one Peltier element, at least one second heat exchanger element (13) through which fluid can flow for transferring heat to or from the fluid and at least one second heat sink (14), and the medical temperature control apparatus (1) is arranged to generate at least one air flow,
- wherein the medical temperature control apparatus (1) is configured such that a first air flow is directed towards the at least one first heat sink (11) of the first temperature control unit (7) and a second air flow is directed towards the at least one second heat sink (14) of the second temperature control unit,
**characterized in that**
the at least one first heat sink (11) and the at least one second heat sink (14) are spatially separated from one another by the pump device (6).

## Revendications

1. Appareil médical de mise en température (1) comprenant,
au moins un boîtier (2),
au moins un dispositif d'alimentation en fluide (3), par lequel un fluide est fourni dans le boîtier (2),
au moins un dispositif de mise en température (4) disposé dans le boîtier (2), qui met en température le fluide s'écoulant à travers le boîtier (2),
au moins un dispositif d'évacuation de fluide (5), par lequel le fluide est guidé à l'extérieur du boîtier (2) à une température sensiblement prédéfinie,
au moins un dispositif de pompage (6) disposé dans le boîtier (2), lequel sollicite le fluide s'écoulant à travers le boîtier (2) en pression, et
que ce au moins un dispositif de mise en température (4) présente au moins une première unité de mise en température (7) et au moins une deuxième unité de mise en température (8),
dans lequel la première unité de mise en température (7) présente au moins un premier élément thermoélectrique (9), en particulier au moins un élément Peltier, au moins un premier élément échangeur de chaleur (10) pouvant être traversé par le fluide pour la transmission de chaleur sur le ou à partir du fluide et au moins un premier corps de refroidissement (11), et
dans lequel la deuxième unité de mise en température (8) présente au moins un deuxième élément thermoélectrique (12), en particulier au moins un élément Peltier, au moins un deuxième élément échangeur de chaleur (13) pouvant être traversé par le fluide pour la transmission de chaleur sur le ou à partir du fluide et au moins un deuxième corps de refroidissement (14), et
l'appareil médical de mise en température (1) est conçu pour produire au moins un flux d'air,
dans lequel un premier flux d'air est dirigé sur l'au moins un premier corps de refroidissement (11) de la première unité de mise en température (7) et un deuxième flux d'air est dirigé sur l'au moins un deuxième corps de refroidissement (14) de la deuxième unité de température (8),
**caractérisé en ce que**
l'au moins un premier corps de refroidissement (11) et l'au moins un deuxième corps de refroidissement (14) sont séparés l'un de l'autre dans l'espace par le dispositif de pompage (6).

2. Appareil médical de mise en température selon la revendication 1, dans lequel
le premier corps de refroidissement (11) de la première unité de mise en température (7) présente une zone avec des nervures de refroidissement (11a) et la zone avec des nervures de refroidissement (11a) de la première unité de mise en température (7) est traversée par le premier flux d'air, et
le deuxième corps de refroidissement (14) de la deuxième unité de mise en température (8) présente une zone avec des nervures de refroidissement (14a) et la zone avec des nervures de refroidissement (14a) de la deuxième unité de mise en température (8) est traversée par le deuxième flux d'air.

3. Appareil médical de mise en température selon la revendication 2, dans lequel
le premier flux d'air passe sensiblement parallèlement aux plans dans lesquels les nervures de refroidissement (11a) respectives du premier corps de refroidissement (11) de la première unité de mise en température (7) s'étendent et lesquels sont disposés perpendiculairement au plan qui est obtenu par la juxtaposition à plat du premier thermoélément (12) et du premier corps de refroidissement (11) et de manière correspondante le deuxième flux d'air passe sensiblement parallèlement aux plans dans lesquels les nervures de refroidissement (14a) respectives du deuxième corps de refroidissement (14) de la deuxième unité de mise en température (8) s'étendent et lesquels sont disposés perpendiculairement au plan qui est obtenu par la juxtaposition directement à plat du deuxième thermoélément (12) et du deuxième corps de refroidissement (14).

4. Appareil médical de mise en température selon la revendication 2, dans lequel
les nervures de refroidissement (11a) du premier corps de refroidissement (11) de la première unité de mise en température (7) et/ou les nervures de refroidissement (14a) du deuxième corps de refroidissement (14) de la deuxième unité de mise en température (8) sont fabriquées dans le procédé « à ailettes très serrées ».

5. Appareil médical de mise en température selon l'une quelconque des revendications précédentes, dans lequel
les corps de refroidissement (11, 14) de la première et de la deuxième unité de mise en température (7, 8) sont fabriqués à partir de cuivre.

6. Appareil médical de mise en température selon l'une quelconque des revendications précédentes, dans lequel
la première unité de mise en température (7) et la deuxième unité de mise en température (8) sont disposées sensiblement avec une symétrie spéculaire l'une par rapport à l'autre.

7. Appareil médical de mise en température selon l'une quelconque des revendications précédentes, dans lequel
la première unité de mise en température (7) présente au moins un premier dispositif de ventilation (15) et la deuxième unité de mise en température (8) présente au moins un deuxième dispositif de ventilation (16).

8. Appareil médical de mise en température selon la revendication 7, dans lequel
l'au moins un premier dispositif de ventilation (15) de la première unité de mise en température (7) génère le premier flux d'air et l'au moins un deuxième dispositif de ventilation (16) de la deuxième unité de mise en température (8) produit le deuxième flux d'air, dans lequel le premier flux d'air du premier dispositif de ventilation (15) est dirigé dans l'intérieur du boîtier (2) et le deuxième flux d'air du deuxième dispositif de ventilation (16) est dirigé dans l'intérieur du boîtier (2).

9. Appareil médical de mise en température selon la revendication 8, dans lequel
l'au moins un premier dispositif de ventilation (15) de la première unité de mise en température (7) est orienté en direction d'au moins une première paroi latérale (17) du boîtier (2) et l'au moins un deuxième dispositif de ventilation (16) de la deuxième unité de mise en température (8) est orienté en direction de la première paroi latérale (17) du boîtier (2), de sorte que le premier flux d'air et ou le deuxième flux d'air parviennent dans l'intérieur du boîtier (2) par au moins une ouverture (18) dans la première paroi latérale (17) du boîtier (2), ou
l'au moins un premier dispositif de ventilation (15) de la première unité de mise en température (7) est orienté en direction d'au moins la première paroi latérale (17) du boîtier (2), de sorte que le premier flux d'air parvient dans l'intérieur du boîtier (2) par l'au moins une ouverture (18) dans la première paroi latérale (17) du boîtier (2) et l'au moins un deuxième dispositif de ventilation (16) de la deuxième unité de mise en température (8) est orienté en direction de l'au moins une deuxième paroi latérale (19) du boîtier (2), de sorte que le deuxième flux d'air parvient dans l'intérieur du boîtier (2) par au moins une ouverture (18') dans la deuxième paroi latérale (19) du boîtier (2).

10. Appareil médical de mise en température selon l'une quelconque des revendications précédentes, dans lequel
le dispositif d'alimentation en fluide (3) présente un dispositif d'amenée de fluide (5), par lequel le fluide est introduit de l'extérieur dans le boîtier (2).

11. Appareil médical de mise en température selon la revendication 10, dans lequel
le dispositif d'amenée de fluide (5) est prévu pour l'introduction dans le boîtier (2) d'au moins une partie du fluide guidé à l'extérieur du boîtier (2) par le dispositif d'évacuation de fluide (20), de sorte qu'un circuit de fluide au moins partiel est obtenu, dans lequel
le fluide introduit dans le boîtier (2) par le dispositif d'amenée de fluide (5) présente une autre température, de préférence plus basse, plus préférablement plus élevée, et/ou une autre pression, de préférence plus baisse, que le fluide guidé à l'extérieur du boîtier (2) par le dispositif d'évacuation de fluide (20).

12. Appareil médical de mise en température selon l'une quelconque des revendications précédentes, dans lequel
le dispositif d'alimentation en fluide (3) présente un réservoir de fluide (21) disposé dans le boîtier (1).

13. Appareil médical de mise en température selon les revendications 10 et 12, dans lequel
le dispositif d'amenée de fluide (5) est en liaison fluidique avec le réservoir de fluide (21).

14. Procédé pour la fabrication d'un appareil médical de mise en température, en particulier d'un appareil de mise en température selon l'une quelconque des revendications précédentes, présentant les étapes :
• de fourniture et disposition d'au moins un boîtier (2),
• de fourniture et disposition d'au moins un dispositif de mise en température (4) disposé dans le boîtier (2), qui met en température le fluide s'écoulant à travers le boîtier (2), dans lequel ce au moins un dispositif de mise en température (4) présente au moins une première unité de mise en température (7) et au moins une deuxième unité de mise en température (8),
dans lequel la première unité de mise en température (7) présente au moins un premier élément thermoélectrique (9), en particulier au moins un élément Peltier, au moins un premier élément échangeur de chaleur (10) pouvant être traversé par un fluide pour la transmission de chaleur sur le ou à partir du fluide et au moins un premier corps de refroidissement (11), et
dans lequel la deuxième unité de mise en température (8) présente au moins un deuxième élément thermoélectrique (12), en particulier au moins un élément Peltier, au moins un deuxième élément échangeur de chaleur (13) pouvant être traversé par un fluide pour la transmission de chaleur sur le ou à partir du fluide et au moins un deuxième corps de refroidissement (14), et l'appareil médical de mise en température (1) est conçu pour produire au moins un flux d'air,
• dans lequel l'appareil médical de mise en température (1) est conçu de sorte qu'un premier flux d'air est dirigé sur l'au moins un premier corps de refroidissement (11) de la première unité de mise en température (7) et un deuxième flux d'air est dirigé sur l'au moins un deuxième corps de refroidissement (14) de la deuxième unité de mise en température,
**caractérisé en ce que**
l'au moins un premier corps de refroidissement (11) et l'au moins un deuxième corps de refroidissement (14) sont séparés l'un de l'autre dans l'espace par le dispositif de pompage (6).
